# EUROPÄISCHE PATENTSCHRIFT

(11) **EP 2 287 122 B1**
(45) Veröffentlichungstag und Bekanntmachung des Hinweises auf die Patenterteilung: **15.05.2013**
(21) Anmeldenummer: 09009626.4
(22) Anmeldetag: 24.07.2009
(51) Int. Cl.: C03C 10/16, A61K 6/027

(54) **Phosphosilikat-Glaskeramik**
Phosphosilicate glass ceramic
Vitrocéramique de phosphosilicate

(43) Veröffentlichungstag der Anmeldung: 23.02.2011
(73) Patentinhaber: Ivoclar Vivadent AG, 9494 Schaan (LI)
(72) Erfinder: Bolle, Urs, 6842 Koblach (AT); Schweiger, Marcel, 7000 Chur (CH); Apel, Elke, 9479 Oberschan (CH); Höland, Wolfram, 9494 Schaan (LI); Rheinberger, Volker, 9490 Vaduz (LI)
(74) Vertreter: UEXKÜLL & STOLBERG

(56) Entgegenhaltungen:
- DE-A1- 10 351 885
- DE-A1- 19 725 555
- DE-B3-102004 013 455
- DE-C1- 4 423 793

## Beschreibung

### Technisches Gebiet

Die Erfindung betrifft eine Phosphosilikat-Glaskeramik, die Fluorapatit-Kristalle und Leucit-Kristalle aufweist, chemisch stabil sowie optisch sehr ansprechend ist und sich daher zur Herstellung von dentalen Restaurationen und insbesondere zum Überpressen von Dentallegierungen eignet.

### Stand der Technik

Phosphosilikat-Glaskeramiken sind aus dem Stand der Technik bekannt. Diese enthalten in manchen Fällen Fluorapatit und/ oder Leucit als Haupt- oder Nebenkristallphase.

DE 44 23 793 beschreibt eine Phosphosilikat-Glaskeramik, die neben einer Leucit-Hauptkristallphase mindestens eine weitere Kristallphase sowie eine oder mehrere Glasphasen enthält. Die Nebenkristallphase kann stäbchen- oder nadelförmige Apatitkristalle, u.a. Fluorapatit aufweisen, die eine Länge von mehr als 2µm haben. Zur Abscheidung von nadelförmigen Fluorapatitkristallen ist allerdings ein hoher Anteil an CaO notwendig. Weiter macht der hohe Leucitgehalt der Glaskeramik relativ hohe Verarbeitungstemperaturen von bis zu 1200°C erforderlich, um die Glaskeramik zur erwünschten Dentalsuprastruktur zu verpressen, d.h. um viskoses Fließen der Glaskeramik zu erreichen. Außerdem bedingt der hohe Leucit-Anteil einen hohen linearen Ausdehnungskoeffizienten im Bereich von ca. 15 bis ca. 20 x 10⁻⁶K⁻¹. Die Glaskeramik ist deshalb zur Beschichtung von Werkstoffen mit niedrigem Ausdehnungskoeffizienten, wie speziellen Metalllegierungen, nicht geeignet.

DE 197 25 555 beschreibt eine transluzente Glaskeramik, deren Hauptkristallphase aus Fluorapatit-Kristallen besteht. Zusätzlich können je nach Zusammensetzung des eingesetzten Ausgangsglases weitere Kristallphasen gebildet werden, jedoch keine Leucit-Phase. Die Glaskeramik hat einen thermischen Ausdehnungskoeffizient von 6.0 bis 12.0 x 10⁻⁶K⁻¹. Dies beschränkt die Anwendung der Glaskeramik, indem sie lediglich zur Beschichtung oder Verblendung von Dentalsuprastrukturen aus Werkstoffe mit sehr niedrigem Ausdehnungskoeffizienten, wie Lithiumdisilikat-Glaskeramiken oder Titan, eingesetzt werden kann. Zudem sind auch sehr hohe Verarbeitungstemperaturen von bis zu 1200°C notwendig, um die Glaskeramik zur erwünschten Dentalstruktur zu verpressen.

DE 103 51 885 beschreibt eine Glaskeramik mit hoher Opaleszenz, die eine Leucit-Kristallphase sowie eine in der kontinuierlichen Glasphase dispergierte diskontinuierliche weitere Glasphase aufweist. Die Glaskeramik kann durch Aufbrennen auf Metalle oder Metalllegierungen zur Herstellung von metallkeramischem Zahnersatz dienen.

### Beschreibung der Erfindung

Der Erfindung liegt die Aufgabe zugrunde, eine Phosphosilikat-Glaskeramik zur Verfügung zu stellen, die trotz sehr geringer Gehalte an CaO Apatit-Kristalle aufweist, die zudem sehr klein sind und insbesondere im nanoskaligen Bereich liegen, und die dadurch in ihren optischen Eigenschaften dem natürlichen Zahnmaterial sehr ähnelt. Weiterhin soll sich die Glaskeramik durch einen niedrigen linearen thermischen Ausdehnungskoeffizienten sowie eine niedrige Presstemperatur auszeichnen und daher insbesondere für das Überpressen von Metalllegierungen geeignet sein.

Überraschenderweise wird diese Aufgabe durch die erfindungsgemäße Phosphosilikat-Glaskeramik gelöst.

Die erfindungsgemäße Phosphosilikat-Glaskeramik ist **dadurch gekennzeichnet, dass** sie die folgenden Komponenten enthält:
SiO₂ 57.6 - 62.0 Gew.%
Al₂O₃ 12.0 - 16.0 Gew.%
B₂O₃ 0 . 0 - 1.5 Gew.%
K₂O 9 . 0 - 13.0 Gew.%
Na₂O 5.0 - 8.0 Gew.%
Li₂O 0.0 - 1.5 Gew.%
BaO 0.0 - 2.5 Gew.%
CaO 0.6 - 2.4 Gew.%
ZnO 0.0 - 3.0 Gew.%
TiO₂ 0.0 - 1.5 Gew.%
ZrO₂ 0.0 - 3.5 Gew.%
CeO₂ 0.0 - 1.0 Gew.%
P₂O₅ 0 . 4 - 2.5 Gew.%
F 0.3 - 1.5 Gew.% und Fluorapatit-Kristalle sowie Leucit-Kristalle aufweist.

Die erfindungsgemäße Glaskeramik zeichnet sich insbesondere dadurch aus, dass in ihr trotz der gegenüber konventionellen Glaskeramiken sehr niedrigen Mengen an CaO überraschenderweise Fluorapatit-Kristalle in der Glasphase vorliegen. Bei den konventionellen Glaskeramiken ist hingegen eine CaO-Menge von mindestens 2,5 Gew.-% erforderlich, um die Bildung von stäbchen- oder nadelförmigen phosphathaltigen Kristallen zu bewirken.

Des Weiteren weist die erfindungsgemäße Glaskeramik im Vergleich zu bekannten Glaskeramiken sehr geringe Mengen an Fluorapatit- und Leucit-Kristallen auf, was zu einer ausgesprochen günstigen Kombination von Eigenschaften führt. Neben hervorragenden optische Eigenschaften besitzt die erfindungsgemäße Glaskeramik einen Wärmeausdehnungkoeffizienten, der sie für eine Vielzahl von Verarbeitungsmöglichkeiten geeignet macht, und sie hat eine niedrige Presstemperatur.

In einer bevorzugten Ausführungsform ist die erfindungsgemäße Phosphosilikat-Glaskeramik **dadurch gekennzeichnet, dass** sie die folgenden Komponenten unabhängig voneinander in Mengen von
SiO₂ 58.0 - 61.5 Gew.%, bevorzugt 58.0 - 61.0 Gew.%
Al₂O₃ 12.0 - 15.0 Gew.%, bevorzugt 13.0 - 14.5 Gew.%
B₂O₃ 0.1 - 1.2 Gew.%, bevorzugt 0.1 - 0.8 Gew.%
K₂O 9.0 - 12.5 Gew.%, bevorzugt 9.0 - 12.0 Gew.%
Na₂O 5.5 - 8.0 Gew.%, bevorzugt 6.0 - 8.0 Gew.%
Li₂O 0.0 - 1.0 Gew.%, bevorzugt 0.1 - 0.8 Gew.%
BaO 0.0 - 2.0 Gew.%, bevorzugt 0.0 - 1.5 Gew.%
CaO 1.0 - 2.4 Gew.%, bevorzugt 1.2 - 2.2 Gew.%
ZnO 0.0 - 2.7 Gew.%, bevorzugt 0.1 - 2.5 Gew.%
TiO₂ 0.2 - 1.5 Gew.%, bevorzugt 0.2 - 1.3 Gew.%
ZrO₂ 0.8 - 3.5 Gew.%, bevorzugt 0.8 - 3.0 Gew.%
CeO₂ 0.2 - 1.0 Gew.%, bevorzugt 0.3 - 0.9 Gew.%
P₂O₅ 0.4 - 2.2 Gew.%, bevorzugt 0.4 - 2.0 Gew.%
F 0.5 - 1.5 Gew.%, bevorzugt 0.6 - 1.5 Gew.%
   enthält.

Der Begriff "unabhängig voneinander" bedeutet, dass wenigstens eine der Komponenten in der angegebenen bevorzugten Menge vorliegt. Beispielsweise kann lediglich der Gehalt von SiO₂ in einer Menge entsprechend der bevorzugten Ausführungsform von 58.0 bis 61.5 Gew.-% vorliegen.

Außerdem ist bevorzugt, dass die Glaskeramik nadelförmige Fluorapatit-Kristalle sowie bevorzugt sehr kleine Fluorapatit-Kristalle aufweist. Dabei sind solche bevorzugt, die eine maximale Ausdehnung von weniger als 500nm, bevorzugt weniger als 250nm und besonders bevorzugt weniger als 100nm haben. Um die maximale Ausdehnung der Kristalle zu bestimmen, wurde deren Länge in c-Achsenrichtung anhand von rasterelektronischen Aufnahmen vermessen. Besonders bevorzugt haben die Fluorapatit-Kristalle eine Länge von weniger als 100nm und eine Breite von nicht mehr als 60nm. Es wurde überraschend festgestellt, dass die Fluorapatitkristalle in der erfindungsgemäßen Glaskeramik keiner Ostwald-Reifung unterliegen, die ansonsten zur Ausbildung größerer Kristalle zu Lasten der Anzahl der kleineren Kristalle führen würde. Insbesondere die kleinen Fluorapatit-Kristalle führen zu den erwünschten optischen Eigenschaften der Glaskeramik, d.h. sie ergeben eine starke optische Ähnlichkeit mit dem natürlichen Zahnmaterial.

Weiter wird überraschenderweise die Transluzenz der erfindungsgemäßen Glaskeramik durch die darin enthaltenen Leucit-Kristalle nicht wesentlich beeinträchtigt. Dies ist vermutlich darauf zurückzuführen, dass die spezielle erfindungsgemäße Zusammensetzung zu einer nur geringen Kristallisation an Leucit führt.

Die erfindungsgemäße Glaskeramik hat bevorzugt einen linearen thermischen Ausdehnungskoeffizienten von 11.0 bis 15.0 x 10⁻⁶K⁻¹, und insbesondere von 11.5 bis 14.0 x 10⁻⁶K⁻¹, gemessen in einem Temperaturbereich von 100 bis 400°C, hat.

Besonders vorteilhaft ist außerdem, dass die erfindungsgemäße Glaskeramik eine niedrige Presstemperatur von üblicherweise unter 1000°C, insbesondere von 850°C bis 950°C aufweist. Dardurch können Dentallegierungen mit ihr überpresst werden, ohne dass es zu einer wesentlichen Verformung der Legierungen kommt. Eine derartige Verformung ist bei der Herstellung von paßgenauen Restaurationen sehr nachteilig.

Der Ausdehnungskoeffizient sowie die Presstemperatur sind wichtige Eigenschaften im Zusammenhang mit der bevorzugten Verwendung der erfindungsgemäßen Glaskeramik zur Herstellung von Dentalrestaurationen. Dabei eignet sich die erfindungsgemäße Glaskeramik insbesondere als Beschichtungsmaterial für Metalllegierungen, die als Gerüstwerkstoffe dienen. Um eine stabile Dentalrestauration zu erhalten, sollte der Ausdehnungskoeffizient der Glaskeramik üblicherweise um etwa 10% geringer sein als der Ausdehnungskoeffizient des Gerüstwerkstoffes. Damit werden hohe Spannungen vermieden, die zu Abplatzen oder Rissen führen können.

Die Erfindung betrifft weiter ein Verfahren zur Herstellung der erfindungsgemäßen Phosphosilikat-Glaskeramik, bei dem
a) ein Glas, welches die erfindungsgemäßen Komponenten enthält, bei Temperaturen von 1500 bis 1550°C erschmolzen;
b) die erhaltenen Glasschmelze unter Bildung eines Glasgranulates in Wasser eingegossen;
c) gegebenenfalls das Glasgranulat zu einem Glaspulver mit einer mittleren Korngröße von weniger als 90 µm, bevorzugt 10 bis 40 µm, zerkleinert; und
d) das Glasgranulat oder das Glaspulver einer Wärmebehandlung in einem Temperaturbereich von 500°C bis 1000°C, für eine Dauer von 30 Minuten bis 6 Stunden, unter Bildung der Glaskeramik unterzogen wird.

In Stufe a) wird zunächst ein Ausgangsglas erschmolzen, indem geeignete Materialien, wie zum Beispiel Carbonate, Oxide und Fluoride, innig miteinander vermischt und auf die angegebenen Temperaturen erhitzt werden.

Dann wird in Stufe b) die erhaltene Glasschmelze durch Eingießen in Wasser abgeschreckt und dadurch zu einem Glasgranulat umgewandelt. Diese Vorgehensweise wird üblicherweise auch als Fritten bezeichnet.

Gegebenenfalls wird das Glasgranulat anschließend in Stufe c) zerkleinert und insbesondere mit üblichen Mühlen auf die gewünschte Korngröße gemahlen.

In Stufe d) wird das Glasgranulat oder gegebenenfalls das Glaspulver einer thermischen Behandlung bei einer Temperatur im Bereich 500°C bis 1000°C, bevorzugt bei etwa 950°C, für eine Dauer von 30 Minuten bis 6 Stunden, bevorzugt etwa 1 Stunde, unterzogen, woraufhin sich die Glaskeramik bildet.

Schließlich betrifft die Erfindung auch die Verwendung der erfindungsgemäßen Glaskeramik als
a) Dentalmaterial oder daraus geformtes Dentalprodukt; oder
b) Bestandteil von Dentalmaterialien oder daraus geformten Dentalprodukten.

Als erfindungsgemäße geformte Dentalprodukte, die einen Gehalt an der erfindungsgemäßen Phosphosilikat-Glaskeramik aufweisen, kommen neben gewünscht geformten Rohlingen insbesondere Dentalrestaurationen, wie z.B. ein Inlay, ein Onlay, eine Brücke, ein Stiftaufbau, eine Verblendung, eine Schale, ein Veneer, eine Facette, eine Füllung, ein Verbinder, eine Krone oder eine Teilkrone infrage. Die Rohlinge haben üblicherweise solche Formen wie sie in konventionellen Pressvorrichtungen, wie Pressöfen, eingesetzt werden, z.B. Plättchen, Blöcke oder Zylinder.

In einer bevorzugten Ausführungsform wird die erfindungsgemäße Phosphosilikat-Glaskeramik zur Herstellung eines geformten Dentalprodukts auf ein Dentalgerüst aufgepresst, wobei das Dentalgerüst bevorzugt auf Basis einer Dentallegierung ist.

Dabei erfolgt das Aufpressen vorzugsweise bei einer Temperatur von weniger als 1000°C und insbesondere bei 850-950°C.

Besonders vorteilhaft sind dabei Dentallegierungen, deren linearer thermischer Ausdehnungskoeffizient denjenigen der erfindungsgemäßen Glaskeramik um etwa 10% übersteigt. Die erfindungsgemäße Phosphosilikat-Glaskeramik eignet sich insbesondere zum Aufpressen auf eine Dentallegierung, welche einen linearen thermischen Ausdehnungskoeffizient von 13.5 bis 15.5 x 10⁻⁶K⁻¹, gemessen in einem Temperaturbereich von 100 bis 400°C hat.

Die Erfindung betrifft daher auch ein Verfahren zur Herstellung eines Dentalprodukts, insbesondere einer Dentalrestauration, bei dem
a) ein Dentalgerüst, insbesondere auf Basis einer Dentallegierung, zur Verfügung gestellt wird, und
b) das Dentalgerüst mit der erfindungsgemäßen Glaskeramik, insbesondere durch Überpressen, beschichtet wird.

Die Erfindung wird nachstehend anhand von Beispielen näher erläutert.

### Beispiele

### Beispiele 1 bis 9: Zusammensetzung, Herstellung und Eigenschaften erfindungsgemäßer Glaskeramiken

Es wurden insgesamt 9 verschiedene erfindungsgemäße Glaskeramiken hergestellt, die die in Tabelle I angegebene Zusammensetzung besaßen.

**Tabelle I: Zusammensetzung erfindungsgemäßer Glaskeramiken (Mengen in Gew.-%)**

| **Beispiele** | **1** | **2** | **3** | **4** | **5** | **6** | **7** | **8** |
|---|---|---|---|---|---|---|---|---|
| SiO₂ | 58.60 | 57.60 | 60.00 | 58.89 | 61.00 | 57.60 | 59.90 | 57.60 |
| K₂O | 10.51 | 11.95 | 9.51 | 9.51 | 12.50 | 10.60 | 9.00 | 10.00 |
| Na₂O | 6.35 | 6.20 | 6.35 | 6.35 | 5.50 | 8.00 | 7.50 | 8.00 |
| Al₂O₃ | 13.87 | 13.95 | 13.75 | 14.30 | 14.50 | 12.90 | 13.50 | 13.00 |
| BaO | 1.83 | 1.82 | 1.82 | 1.85 | 1.00 | 2.00 | - | 2.10 |
| CaO | 1.87 | 1.87 | 1.87 | 1.87 | 2.40 | 2.20 | 1.50 | 2.30 |
| P₂O₅ | 0.46 | 0.46 | 0.46 | 0.60 | 0.80 | 0.70 | 0.40 | 0.70 |
| B₂O₃ | 0.10 | 0.10 | 0.10 | 0.10 | - | 0.50 | 0.20 | 0.50 |
| TiO₂ | 0.61 | 0.59 | 0.63 | 0.63 | - | - | 1.50 | - |
| ZrO₂ | 1.36 | 1.20 | 1.30 | 1.40 | 0.80 | 1.00 | 3.50 | 1.00 |
| ZnO | 2.58 | 2.40 | 2.35 | 2.64 | - | 3.00 | 1.50 | 3.00 |
| CeO₂ | 0.76 | 0.76 | 0.76 | 0.76 | 1.00 | - | 0.60 | 0.30 |
| F | 1.10 | 1.10 | 1.10 | 1.10 | 0.50 | 1.50 | 0.90 | 1.50 |
| Phasen | FAP*/ Leucit | FAP*/ Leucit | FAP*/ Leucit | FAP*/ Leucit | FAP*/ Leucit | FAP*/ Leucit | FAP*/ Leucit | FAP*/ Leucit |

| | | | | | | | | |
|---|---|---|---|---|---|---|---|---|
| *: FAP = Fluorapatit | | | | | | | | |

Zur Herstellung der Glaskeramiken wurde die jeweilige Zusammensetzung aus geeigneten Oxiden, Carbonaten und Fluoriden in einem Platin/Rhodium-Tiegel bei einer Temperatur von ungefähr 1500°C bis 1550°C und während einer Homogenisierungszeit von 1 Stunde erschmolzen. Die Glasschmelze wurde in Wasser abgeschreckt, und das gebildete Granulat des Ausgangsglases wurde getrocknet und auf eine mittlere Korngröße von weniger als 90µm aufgemahlen. Anschließend wurde das Granulat oder das erhaltene Pulver des Ausgangsglases 30 Minuten bis 6 Stunden einer ein- oder mehrstufigen Wärmebehandlung bei mehr als 500°C und bis zu 1000°C unterworfen, woraufhin sich die Glaskeramik bildete.

Für einige der Glaskeramiken sind in Tabelle II ausgewählte Eigenschaften angegeben, die an Probekörpern aus der jeweiligen Glaskeramik bestimmt worden sind. Weiter finden sich in Tabelle II unter "Thermischer Behandlung" Angaben zu der konkret gewählten Wärmebehandlung des Ausgangsglases Die Beispiele verdeutlichen, wie durch Veränderung der chemischen Zusammensetzung Glaskeramiken mit unterschiedlichen Eigenschaften erhalten werden können.

**Tabelle II: ausgewählte chemisch-physikalische Eigenschaften erfindungsgemäßer Glaskeramiken**

| **Beispiele** | **2** | **4** | **6** | **9** |
|---|---|---|---|---|
| Thermische Behandlung [°C/Std] | 950/1 | 950/1 | 950/1 | 950/1 |
| Herstellung von Rohlingen durch Sinterung bei [°C/Std] | | 880/0.5 | | 810/0.5 |
| α-Wert Sinterglaskeramik* [x 10⁻⁶K⁻¹] 100-400°C | 15.0 | 9.6 | 13.2 | 12.0 |
| Tg Sinterglaskeramik [°C] | 472 | 566 | 478 | 490 |
| Presstemperatur [°C] | | 950 | | 880 |
| α-Wert verpresster Sinterrohling ** [x 10⁻⁶K⁻¹] 100-400°C | | 11.8 | | 13.1 |
| Tg Presskeramik [°C] | | 547 | | 477 |
| Biaxialfestigkeit [MPa] | | 102±18 | | |
| Essigsäurelöslichkeit [µg/cm²] | | 12 | | |
| Salivalöslichkeit [µg/cm²] | | 20 | | |

| | | | | |
|---|---|---|---|---|
| *: Sinterglaskeramik hergestellt nach unten beschriebenem Verfahren A); **: Pressglaskeramik hergestellt nach unten beschriebenem Verfahren B); | | | | |

### Bestimmung des Ausdehnungskoeffizienten α

### A) Sinterglaskeramik

Zur Messung des linearen thermischen Ausdehnungskoeffizienten α wurde aus dem Pulver der jeweiligen Glaskeramik ein stäbchenförmiger Grünkörper hergestellt, der in einem Vakuumbrennofen mit einer Aufheizrate von 60°C/min und einer Haltezeit von 1 Minute bei der jeweiligen Brenntemperatur gesintert wurde. Anschließend wurde ein Glanzbrand ohne Vakuum bei einer um 20°C höheren Endtemperatur und einer Haltezeit von 1 Minute durchgeführt.

### B) Pressglaskeramik

Über den Sinterprozess wurden auch monolithische Sinterrohlinge hergestellt. Dazu wurde das Ausgangspulver mittels einer Uniaxialpresse mit 500 bis 1000 bar Druck zu einem Grünling gepresst und anschließend unter Vakuum durchgesintert. Die Sinterrohlinge wurden in einem Heisspressofen bei 850 bis 950°C und einem Pressdruck von 19 bis 22 bar in die Prüfgeometrie gepresst.

Der lineare thermische Ausdehnungskoeffizient α wurde in den Beispielen 2, 4, 6 und 8 an gesinterten Glaskeramikprüfkörpern gemäß A) und in den Beispielen 4 und 8 auch an heissgepressten Glaskeramikprüfkörpern gemäß B) in einem Temperaturbereich von 100 bis 400°C bestimmt.

### Bestimmung der Säure- und Salivabeständigkeit

Die Säurebeständigkeit ist ein Maß für die chemischen Beständigkeit gerade von im Dentalbereich eingesetzten Gläsern und Glaskeramiken, da diese in der Mundhöhle permanent der Einwirkung von sauren Substanzen ausgesetzt sind. Die Säurebeständigkeit wurde gemäß der ISO-Vorschrift 6872:1995 bestimmt. Dazu wurden zunächst Probeplättchen mit einem Durchmesser von 12mm und einer Dicke von 1mm durch das Zusammensintern von Glaskeramikpulver mit einer mittleren Teilchengröße von weniger als 90µm hergestellt. Das Pulver wurde 1 Minute lang auf der Sintertemperatur gehalten. Dann wurden die Probeplättchen 16 Stunden lang in einer Soxhlet-Apparatur mit 4 Vol.-%iger wässriger Essigsäure bei 80°C behandelt. Bei dem Salivatest wurden die Prüfkörper für 7 Tage bei 60°C in Kunstspeichel gelagert. Schließlich wurde der eingetretene Masseverlust als Maß für die Säurebeständigkeit bestimmt.

### Bestimmung der Biaxialfestigkeit

Die Biaxialfestigkeit wurde gemäß ISO 6872 bestimmt. Dazu wurden Probeplättchen mit einem Durchmesser von 13mm und einer Dicke von 1.2mm durch das Zusammensintern von Glaskeramikpulver mit einer mittleren Teilchengröße von weniger als 90µm hergestellt. Das Pulver wurde 1 Minute lang auf der Sintertemperatur gehalten. Die Plättchen wurden gemäß Vorschrift ISO 6872 oberflächlich beschliffen und in einer Biaxialprüfapparatur gemessen.

### Beispiel 9 Herstellung einer Schichtkeramik durch Aufpressen der erfindungsgemäßen Glaskeramik auf ein opakisiertes Legierungsgerüst.

In diesem Beispiel wird die Herstellung einer erfindungsgemäßen Glaskeramik mit der Zusammensetzung gemäß Beispiel 8 beschrieben, die in vorteilhafter Weise auf ein opakisiertes Legierungsgerüst aufgepresst werden kann. Hierzu wurde zunächst die Dentallegierung mit einem glaskeramischen Opaker beschichtet, um die metallisch dunkle Farbe abzudecken. Dann wurde ein Ausgangsglas mit der in Tabelle I für Beispiel 9 angegebenen chemischen Zusammensetzung hergestellt. Zur Herstellung wurde ein entsprechendes Gemenge von geeigneten Oxiden, Carbonaten und Fluoriden in einem Platin/Rhodium-Tiegel bei einer Temperatur von 1500°C während einer Homogenisierungszeit von 1 Std. erschmolzen. Die Glasschmelze wurde in Wasser abgeschreckt, und das gebildete Granulat des Ausgangsglases wurde getrocknet und auf eine mittlere Korngröße von weniger als 90µm, bevorzugt auf eine mittlere Korngröße von 10 bis 30 µm aufgemahlen. Das erhaltene Glaspulver wurde anschließend einer Temperaturbehandlung von 950°C während 1 Stunde unterzogen. Die Glaskeramik wurde anschließend auf eine mittlere Korngröße von weniger als 90 µm aufgemahlen, bevorzugt auf eine mittlere Korngröße von 20 bis 40 µm.

Dieses Pulver wurde zu einem stäbchenförmigen Grünkörper in einem Vakuumofen bei einer Aufheizgeschwindigkeit von 60°C/min und einer Haltezeit von 1 Minute bei 900°C gesintert. Für die so erhaltene Probe wurde ein thermischer Ausdehnungskoeffizient von 12,0 x 10⁻⁶K⁻¹, gemessen im Temperaturbereich von 100°C bis 400°C, bestimmt.

Das Glaskeramikpulver wurde bei 810°C während 15 min Haltezeit und Vakuum zu Sinterrohlingen gebrannt. Diese Rohlinge wurden anschließend durch einen Heißpressprozess bei 880°C in stäbchenförmige Prüfkörper gepresst. Für die so erhaltene Glaskeramikprobe wurde ein Ausdehnungskoeffizient von 13,1 x 10⁻⁶K⁻¹, gemessen im Temperaturbereich von 100°C bis 400°C, bestimmt.

Diese Glaskeramik eignet sich daher besonders gut zum Aufpressen auf opakisierte Dentallegierungen. Es wurden hierfür zwei Dentallegierungen getestet, d.Sign 30 (Ivoclar Vivadent AG) mit einem Ausdehnungskoeffizienten von 14,5 x 10⁻⁶ K⁻¹ und W1 (Ivoclar Vivadent AG) mit einem Ausdehnungskoeffizienten von 15,2 x 10⁻⁶K⁻¹. Es wurden aus beiden Legierungen Molarengerüste nach Vorgaben des Legierungsherstellers hergestellt. Die Gerüste wurden mit IPS InLine PoM Opaker (Ivoclar Vivadent AG) gemäß Herstellerangaben opakiert. Danach wurden die Metallgerüste aufgewachst, um die anatomische Form des Zahnes abzubilden. Die so vorbereiteten Metallgerüste wurden mit Press Vest Speed Einbettmasse eingebettet und das Wachs wurde ausgebrannt. Die Sinterrohlinge der Glaskeramik wurden bei 900°C und einem Druck von 19 bis 22 bar in die Hohlform gepresst. Durch den angepassten Ausdehnungskoeffizienten und die angepasste Verarbeitungstemperatur ließ sich über den Heißpressprozess ein fester Werkstoffverbund zwischen opakisierter Dentallegierung und Glaskeramik erzielen. Nach dem Auskühlen und Ausbetten durch Sandstrahlen waren die Molarenkronen vollständig ausgepresst und rissfrei. Um die zahntechnische Handhabung zu vervollständigen, wurden die Kronen jeweils zweimal einem Glasurbrand mit IPS InLine PoM-Glasur (Ivoclar Vivadent AG) bei 770°C unterzogen. Auch nach diesem zusätzlichen thermischen Zyklen waren die Kronen rissfrei.

### Beispiel 10 Gefügebild der Glaskeramik

Um das Gefüge einer erfindungsgemäßen Glaskeramik rasterelektronenmikroskopisch darzustellen, wurde zunächst eine Glaskeramik mit der Zusammensetzung gemäß Beispiel 3 nach thermischer Behandlung von 1 Stunde bei 950°C erzeugt. Ein rasterelektronenmikroskopisches Bild wurde nach Probenpräparation durch Ätzen mit 3%iger wässriger HF-Lösung für eine Dauer von 10 Sekunden erstellt. Durch diesen Ätzvorgang wurde die SiO₂-reiche Glasmatrix in einer dünnen Schicht aufgelöst, so dass insbesondere die Fluorapatit-Kristalle aus der Probenebene heraustraten und somit deutlich sichtbar wurden. Diese Kristalle lagen isoliert voneinander vor und besaßen eine Länge von ca. 100nm.

## Patentansprüche

1. Phosphosilikat-Glaskeramik, **dadurch gekennzeichnet, dass** sie die folgenden Komponenten enthält:
SiO₂ 57.6 - 61.5 Gew.%
Al₂O₃ 12.0 - 16.0 Gew.%
B₂O₃ 0 . 0 - 1.5 Gew.%
K₂O 9 . 0 - 13.0 Gew.%
Na₂O 5.0 - 8.0 Gew.%
Li₂O 0.0 - 1.5 Gew.%
BaO 0.0 - 2.5 Gew.%
CaO 0.6 - 2.4 Gew.%
ZnO 0.0 - 3.0 Gew.%
TiO₂ 0.0 - 1.5 Gew.%
ZrO₂ 0.0 - 3.5 Gew.%
CeO₂ 0.0 - 1.0 Gew.%
P₂O₅ 0.4 - 2.5 Gew.%
F 0.3 - 1.5 Gew.%
und Fluorapatit-Kristalle sowie Leucit-Kristalle aufweist.

2. Glaskeramik nach Anspruch 1, **dadurch gekennzeichnet, dass** sie die folgenden Komponenten unabhängig voneinander in Mengen von
SiO₂ 58.0 - 61.5 Gew.%, bevorzugt 58.0 - 61.0 Gew.%
Al₂O₃12.0 - 15.0 Gew.%, bevorzugt 13.0 - 14.5 Gew.%
B₂O₃ 0.1 - 1.2 Gew.%, bevorzugt 0.1 - 0.8 Gew.%
K₂O 9.0 - 12.5 Gew.%, bevorzugt 9.0 - 12.0 Gew.%
Na₂O 5.5 - 8.0 Gew.%, bevorzugt 6.0 - 8.0 Gew.%
Li₂O 0.0 - 1.0 Gew.%, bevorzugt 0.1 - 0.8 Gew.%
BaO 0.0 - 2.0 Gew.%, bevorzugt 0.0 - 1.5 Gew.%
CaO 1.0 - 2.4 Gew.%, bevorzugt 1.2 - 2.2 Gew.%
ZnO 0.0 - 2.7 Gew.%, bevorzugt 0.1 - 2.5 Gew.%
TiO₂ 0.2 - 1.5 Gew.%, bevorzugt 0.2 - 1.3 Gew.%
ZrO₂ 0.8 - 3.5 Gew.%, bevorzugt 0.8 - 3.0 Gew.%
CeO₂ 0.2 - 1.0 Gew.%, bevorzugt 0.3 - 0.9 Gew.%
P₂O₅ 0.4 - 2.2 Gew.%, bevorzugt 0.4 - 2.0 Gew.%
F 0.5 - 1.5 Gew.%, bevorzugt 0.6 - 1.5 Gew.%
enthält.

3. Glaskeramik nach Anspruch 1 oder 2, **dadurch gekennzeichnet, dass** die Fluorapatit-Kristalle nadelförmig sind.

4. Glaskeramik nach einem der Ansprüche 1 bis 3, **dadurch gekennzeichnet, dass** die Fluorapatit-Kristalle eine maximale Ausdehnung von weniger als 500nm, bevorzugt weniger als 250nm und besonders bevorzugt weniger als 100nm haben.

5. Glaskeramik nach einem der Ansprüche 1 bis 4, **dadurch gekennzeichnet, dass** sie einen linearen thermischen Ausdehnungskoeffizienten von 11.0 bis 15.0 x 10⁻⁶K⁻¹, bevorzugt von 11.5 bis 14.0 x 10^{-6K-1}, gemessen in einem Temperaturbereich von 100 bis 400°C, hat.

6. Glaskeramik nach einem der Ansprüche 1 bis 5, **dadurch gekennzeichnet, dass** sie eine Presstemperatur von weniger als 1000°C, bevorzugt von 850°C bis 950°C hat.

7. Verfahren zur Herstellung der Phosphosilikat-Glaskeramik nach einem der Ansprüche 1 bis 6, **dadurch gekennzeichnet dass**
a) ein Glas, welches die Komponenten gemäß Anspruch 1 enthält, bei Temperaturen von 1500 bis 1550°C erschmolzen wird;
b) die erhaltenen Glasschmelze unter Bildung eines Glasgranulates in Wasser eingegossen wird;
c) gegebenenfalls das Glasgranulat zu einem Glaspulver mit einer mittleren Korngröße von weniger als 90 µm, bevorzugt 10 bis 40 µm, zerkleinert wird; und
d) das Glasgranulat oder das Glaspulver einer Wärmebehandlung in einem Temperaturbereich von 500°C bis 1000°C, für eine Dauer von 30 Minuten bis 6 Stunden, unter Bildung der Glaskeramik unterzogen wird.

8. Verfahren nach Anspruch 7, **dadurch gekennzeichnet dass** die Wärmebehandlung bei einer Temperatur von etwa 950°C, für eine Dauer von etwa 1 Stunde durchgeführt wird.

9. Verwendung der Glaskeramik nach einem der Ansprüche 1 bis 6 als
a) Dentalmaterial oder daraus geformtes Dentalprodukt; oder
b) Bestandteil von Dentalmaterialien oder daraus geformten Dentalprodukten.

10. Verwendung nach Anspruch 9, bei der die Glaskeramik auf ein Dentalgerüst aufgepresst wird.

11. Verwendung nach Anspruch 10, wobei das Aufpressen bei einer Temperatur von weniger als 1000°C und insbesondere bei 850 bis 950°C erfolgt.

12. Verwendung nach Anspruch 10 oder 11, bei der das Dentalgerüst auf Basis einer Dentallegierung ist.

13. Verwendung nach Anspruch 12, wobei die Dentallegierung einen linearen Ausdehnungskoeffizienten von 13.5 bis 15.5 x 10⁻⁶K⁻¹, gemessen in einem Temperaturbereich von 100 bis 400°C, besitzt.

## Claims

1. Phosphosilicate glass ceramic **characterized in that** it comprises the following components:
SiO₂ 57.6 - 61.5 wt.-%
Al₂O₃ 12.0 - 16.0 wt.-%
B₂O₃ 0.0 - 1.5 wt.-%
K₂O 9.0 - 13.0 wt.-%
Na₂O 5.0 - 8.0 wt.-%
Li₂O 0.0 - 1.5 wt. -%
BaO 0.0 - 2.5 wt.-%
CaO 0.6 - 2.4 wt.-%
ZnO 0.0 - 3.0 wt.-%
TiO₂ 0.0 - 1.5 wt.-%
ZrO₂ 0.0 - 3.5 wt.-%
CeO₂ 0.0 - 1.0 wt.-%
P₂O₅ 0.4 - 2.5 wt.-%
F 0.3 - 1.5 wt.-%
and comprises fluoroapatite, crystals and leucite crystals.

2. Glass ceramic according to claim 1 **characterized in that** it comprises the following components independently of each other in amounts of
SiO₂ 58.0- 61.5 wt.-%, preferably 58.0- 61.0 wt.-%
Al₂O₃ 12.0- 15.0 wt.-%, preferably 13.0- 14.5 wt.-%
B₂O₃ 0.1 - 1.2 wt.-%, preferably 0.1 - 0.8 wt.-%
K₂O 9.0 - 12.5 wt.-%, preferably 9.0 - 12.0 wt.-%
Na₂O 5.5 - 8.0 wt.-%, preferably 6.0 - 8.0 wt.-%
Li₂O 0.0 - 1.0 wt.-%, preferably 0.1 - 0.8 wt.-%
BaO 0.0 - 2.0 wt.-%, preferably 0.0 - 1.5 wt.-%
CaO 1.0 - 2.4 wt.-%, preferably 1.2 - 2.2 wt.-%
ZnO 0.0 - 2.7 wt.-%, preferably 0.1 - 2.5 wt.-%
TiO₂ 0.2 - 1.5 wt.-%, preferably 0.2 - 1.3 wt.-%
ZrO₂ 0.8 - 3.5 wt.-%, preferably 0.8 - 3.0 wt.-%
CeO₂ 0.2 - 1.0 wt.-%, preferably 0.3 - 0.9 wt.-%
P₂O₅ 0.4 - 2.2 wt.-%, preferably 0.4 - 2.0 wt.-%
F 0.5 - 1.5 wt.-%, preferably 0.6 - 1.5 wt.-%.

3. Glass ceramic according to claim 1 or 2, **characterized in that** the fluoroapatite crystals are needle-shaped.

4. Glass ceramic according to any one of claims 1 to 3, **characterized in that** the fluoroapatite crystals have a maximum extension of less than 500 nm, preferably less than 250 nm and particularly preferably less than 100 nm.

5. Glass ceramic according to any one of claims 1 to 4, **characterized in that** it has a linear thermal expansion coefficient of 11.0 to 15.0 x 10⁻⁶K⁻¹, preferably 11.5 to 14.0 x 10⁻⁶K⁻¹, measured in a temperature range of 100 to 400°C.

6. Glass ceramic according to any one of claims 1 to 5, **characterized in that** it has a pressing temperature of less than 1000°C, preferably 850°C to 950°C.

7. Process for the preparation of the phosphosilicate glass ceramic according to any one of claims 1 to 6, comprising
a) melting a glass comprising the components according to claim 1 at temperatures of 1500 to 1550°C;
b) pouring the glass melt obtained into water to form a glass granulate;
c) optionally comminuting the glass granulate to a glass powder with an average particle size of less than 90 µm, preferably 10 to 40 µm; and
d) subjecting the glass granulate or the glass powder to a heat treatment in a temperature range of 500°C to 1000°C for a period of 30 minutes to 6 hours to form the glass ceramic.

8. Process according to claim 7, **characterized in that** the heat treatment is carried out at a temperature of about 950°C for a period of about 1 hour.

9. Use of the glass ceramic according to any one of claims 1 to 6 as
a) a dental material or a dental product shaped therefrom; or
b) a constituent of dental materials or of dental products shaped therefrom.

10. Use according to claim 9, wherein the glass ceramic is pressed onto a dental framework.

11. Use according to claim 10, wherein the pressing on takes place at a temperature of less than 1000°C and in particular at 850 to 950°C.

12. Use according to claim 10 or 11, wherein the dental framework is based on a dental alloy.

13. Use according to claim 12, wherein the dental alloy has a linear expansion coefficient of 13.5 to 15.5 x 10⁻⁶K⁻¹, measured in a temperature range of 100 to 400°C.

## Revendications

1. Vitrocéramique de phosphosilicate, **caractérisée en ce qu'**elle contient les composants suivants :
SiO₂ 57,6 à 61,5% en poids
Al₂O₃ 12,0 à 16,0 % en poids
B₂O₃ 0,0 à 1,5% en poids
K₂O 9,0 à 13,0 % en poids
Na₂O 5,0 à 8,0 % en poids
Li₂O 0,0 à 1,5 % en poids
BaO 0,0 à 2,5 % en poids
CaO 0,6 à 2,4 % en poids
ZnO 0,0 à 3,0 % en poids
TiO₂ 0,0 à 1,5% en poids
ZrO₂ 0,0 à 3,5 % en poids
CeO₂ 0,0 à 1,0 % en poids
P₂O₅ 0,4 à 2,5 % en poids
F 0,3 à 1,5% en poids
et présente des cristaux de fluoroapatite, ainsi que des cristaux de leucite.

2. Vitrocéramique selon la revendication 1, **caractérisée en ce qu'**elle contient les composants suivants indépendamment les uns des autres en des quantités de
SiO₂ 58,0 à 61,5 % en poids, de préférence 58,0 à 61,0 % en poids
Al₂O₃ 12,0 à 15,0 % en poids, de préférence 13,0 à 14,5 % en poids
B₂O₃ 0,1 à 1,2 % en poids, de préférence 0,1 à 0,8 % en poids
K₂O 9,0 à 12,5 % en poids, de préférence 9,0 à 12,0 % en poids
Na₂O 5,5 à 8,0 % en poids, de préférence 6,0 à 8,0 % en poids
Li₂O 0,0 à 1,0 % en poids, de préférence 0,1 à 0,8 % en poids
BaO 0,0 à 2,0 % en poids, de préférence 0,0 à 1,5% en poids
CaO 1,0 à 2,4 % en poids, de préférence 1,2 à 2,2 % en poids
ZnO 0,0 à 2,7 % en poids, de préférence 0,1 à 2,5 % en poids
TiO₂ 0,2 à 1,5 % en poids, de préférence 0,2 à 1,3 % en poids
ZrO₂ 0,8 à 3,5% en poids, de préférence 0,8 à 3,0 % en poids
CeO₂ 0,2 à 1,0 % en poids, de préférence 0,3 à 0,9 % en poids
P₂O₅ 0,4 à 2,2 % en poids, de préférence 0,4 à 2,0 % en poids
F 0,5 à 1,5 % en poids, de préférence 0,6 à 1,5 % en poids.

3. Vitrocéramique selon la revendication 1 ou 2, **caractérisée en ce que** les cristaux de fluoroapatite sont aciculaires.

4. Vitrocéramique selon l'une quelconque des revendications 1 à 3, **caractérisée en ce que** les cristaux de fluoroapatite présentent une expansion maximale inférieure à 500 nm, de préférence inférieure à 250 nm et de manière particulièrement préférée inférieure à 100 nm.

5. Vitrocéramique selon l'une quelconque des revendications 1 à 4, **caractérisée en ce qu'**elle présente un coefficient d'expansion thermique linéaire de 11,0 à 15,0 x 10⁻⁶ K⁻¹, de préférence de 11,5 à 14,0 x 10⁻⁶ K⁻¹, mesuré dans une plage de température allant de 100 à 400 °C.

6. Vitrocéramique selon l'une quelconque des revendications 1 à 5, **caractérisée en ce qu'**elle présente une température de compression inférieure à 1 000 °C, de préférence de 850 °C à 950 °C.

7. Procédé de fabrication de la vitrocéramique de phosphosilicate selon l'une quelconque des revendications 1 à 6, **caractérisé en ce que**
a) un verre, qui contient les composants selon la revendication 1, est fondu à des températures de 1 500 à 1 550 °C ;
b) le verre fondu obtenu est versé dans de l'eau pour former un granulat de verre ;
c) le granulat de verre est éventuellement réduit en une poudre de verre d'une taille de particule moyenne inférieure à 90 µm, de préférence de 10 à 40 µm ; et
d) le granulat de verre ou la poudre de verre est soumis à un traitement thermique dans une plage de température allant de 500 °C à 1 000 °C, pendant une durée de 30 minutes à 6 heures, pour former la vitrocéramique.

8. Procédé selon la revendication 7, **caractérisé en ce que** le traitement thermique est réalisé à une température d'environ 950 °C, pendant une durée d'environ 1 heure.

9. Utilisation de la vitrocéramique selon l'une quelconque des revendications 1 à 6, en tant que
a) matériau dentaire ou produit dentaire formé à partir de celui-ci ; ou
b) constituant de matériaux dentaires ou de produits dentaires formés à partir de ceux-ci.

10. Utilisation selon la revendication 9, dans laquelle la vitrocéramique est comprimée sur une structure dentaire.

11. Utilisation selon la revendication 10, dans laquelle la compression a lieu à une température inférieure à 1 000 °C et notamment de 850 à 950 °C.

12. Utilisation selon la revendication 10 ou 11, dans laquelle la structure dentaire est à base d'un alliage dentaire.

13. Utilisation selon la revendication 12, dans laquelle l'alliage dentaire présente un coefficient d'expansion linéaire de 13,5 à 15,5 x 10⁻⁶ K⁻¹, mesuré dans une plage de température allant de 100 à 400 °C.
